# EUROPEAN PATENT SPECIFICATION

(11) **EP 1 901 723 B1**
(45) Date of publication and mention of the grant of the patent: **23.02.2011**
(21) Application number: 06764872.5
(22) Date of filing: 27.06.2006
(51) Int. Cl.: A61K 9/70, A61P 31/00

(54) **ANTIMICROBIAL MATERIALS**
ANTIMIKROBIELLE MATERIALIEN
SUBSTANCES ANTIMICROBIENNES

(30) Priority: 27.06.2005 GB 0512915; 27.06.2005 GB 0513133
(43) Date of publication of application: 26.03.2008
(73) Proprietor: Smith & Nephew, PLC, London WC2N 6LA (GB)
(72) Inventor: GREENER, Bryan, Elvington,York YO42 4BG (GB)
(74) Representative: Connors, Martin L.H.
(86) International application number: PCT/GB2006/002365
(87) International publication number: WO 2007/000591

(56) References cited:
- EP-A1- 0 528 191
- WO-A2-01/43788
- US-A- 5 395 651
- US-A- 5 837 275
- US-A1- 2002 192 298
- US-A1- 2003 072 810

## Description

This invention relates to compositions comprising materials for the treatment or prophylaxis of microbial, including bacterial, infection, in particular antimicrobial silver species, to some of such materials, to medical devices comprising these materials or compositions, to processes for the provision of such materials, compositions and devices, and to a use in the treatment or prophylaxis of microbial, including bacterial, infections using such materials, compositions or devices.

The clinical antimicrobial activity and efficacy of silver metal and silver compounds is well known. The activity of such metal-based antimicrobial, including antibacterial, materials is due to the release of metal-based species that are soluble, often in water, that are delivered to the area to be treated. For medical device applications, a profile of release spanning several days is needed.

Metal-based materials for the treatment or prophylaxis of microbial, including bacterial, infection exhibit a range of profile of release. Thus, the delivery rate (solubilisation) of silver species from silver metal, for example into aqueous media, is very low indeed. To increase the rate of silver solubilisation, silver salts have been employed, for example silver nitrate treatment. However, silver nitrate is highly soluble in water, and for medical device applications spanning several days, immediate solubility is not desirable.

Silver sulfadiazine does not dissolve immediately in the topical biological environment in which it is applied and has a profile of release spanning several days. However, in these silver salts the presence of a counterion effectively dilutes the quantity of silver that can be provided in a given mass of material (63.5% of the total mass is silver in silver nitrate, only 30.2% in silver sulfadiazine).

The in vitro antimicrobial efficacy of silver oxides has recently attracted commercial interest. Their efficacy can exceed that of traditional silver(I) compounds, and the presence of a counterion of low mass, such as O²⁻ results in less dilution of the quantity of silver that can be provided in a given mass of material.

However, antimicrobial, including antibacterial, metal oxides (and silver(I) salts) suffer from inherent structural instability and/or photosensitivity, and this leads to poor storage stability and poor device compatibility, limiting their medical exploitation.

In addition, use of silver(I) oxide on a substrate of a material containing a nitrogen or sulphur-based group or an oxidisable species, such as a polyurethane, can result in significant degradation of the silver(I) oxide. Polyurethanes based materials are often the substrate of choice in, e.g. dressings, including topical dressings for the management of wounds, and in catheters, stents, drains and some hospital equipment.

A conventional approach to enhancing the stability and ensuring the antimicrobial/ antibacterial activity of metal oxides is complexation of individual metal atoms or ions within the metal oxide. The ligands needed to generate the relevant metal complex and/or the process for their preparation are often complex and/or costly.

Polyanions such as poly(vinylsulphate) and sodium poly(phosphate) are employed to physically stabilise metal nanoclusters, in particular in colloidal aggregates, that are chemically stable under normal ambient conditions. They have not been used in medical applications.

It would be desirable to provide a material for the treatment or prophylaxis of microbial, including bacterial, infection that overcomes the limitations of known antimicrobial, including antibacterial, materials, i.e. it has a profile of release spanning several days, its efficacy exceeds that of traditional silver(I) salts, the presence of a counterion effectively dilutes the quantity of active metal species that can be provided in a given mass of material relatively little, it is stable under normal ambient conditions; particles in sizes ranging from atomic clusters to macroparticles, rather than individual metal atoms or ions within the metal oxide, can be readily stabilised with species that are not complex and/or costly; and can make a substrate of a material containing a nitrogen or sulphur-based group or an oxidisable species, such as a polyurethane, compatible with the metal oxide.

It would also be desirable to provide compositions and devices comprising these materials, processes for the provision of such materials, compositions and devices, and a use in the treatment or prophylaxis of microbial, including bacterial, infections using such materials, compositions or devices.

According to a first aspect of the present invention, there is provided a material for the treatment or prophylaxis of microbial, including bacterial, infections, comprising a metal species and a polymer, wherein the polymer is a polyanion which stabilises the metal species, and wherein the polyanion is polyphosphate.

The metal species may be silver, copper, zinc, manganese, gold, iron, nickel, cobalt, cadmium, palladium and/or platinum species.

The metal species may be a metal ion, metal salt, metal cluster, metal particle, metal nanoparticle and/or metal crystal.

The metal species may be a metal oxide.

The metal species may be a silver species. The metal species may be a silver cation. The silver species may be silver nitrate, silver perchlorate, silver acetate, silver tetrafluoroborate, silver triflate, silver fluoride, silver oxide and/or silver hydroxide. The silver oxide may be silver (I) oxide, silver (I,III) oxide, silver (II,III) oxide, and/or silver (III) oxide.

The metal species may be a gold species. The gold species may be gold nitrate, gold perchlorate, gold acetate, gold tetrafluoroborate, gold triflate, gold fluoride, gold oxide and/or gold hydroxide.

The inorganic polyanion is a polyphosphate.

According to a second aspect of the present disclosure, not part of the invention there is provided a material for the treatment or prophylaxis of microbial, including bacterial, infections, characterised in that it comprises at least one Group IA or IB metal oxide and an organic polyacid and/or a derivative thereof.

When used herein the term 'organic polyacid' means an organic species having a plurality of acid groups, and derivative thereof' means that such groups are derivatised, e.g. by being esterified or an acid salt.

The materials of the first and second aspects of the present invention overcome the limitations of known antimicrobial, including antibacterial, materials. For example, they have a profile of release spanning several days. The materials exhibit a range of profile of release and delivery rate of the relevant active species, for example into aqueous media.

The material compositions and components can be tailored to generate specific desired release rates, for example in aqueous media.

The quantity of silver that can be provided in a given mass of material is effectively diluted relatively little by the presence of the oxide ion or the organic polyacid derivative.

The metal oxide-organic polyacid derivative materials of the first and second aspects of this invention exhibit enhanced stability compared with that of the corresponding metal oxide alone. Articles comprising them can be stored for long periods (up to several years) at ambient temperature and pressure in traditional sterile packaging.

Particles in sizes ranging from atomic clusters to macroparticles, rather than individual metal atoms or ions within the metal oxide, can be readily stabilised with species that are not complex and/or costly.

Effectively a coating of the polyacid derivative complex is formed on the surface of the metal oxide particles.

The metal oxide within the material will usually be in the form of particles in sizes ranging from atomic clusters, nanoclusters, colloids, aggregates, nanoparticles and microparticles, for example to macroparticles, with levels of structural order ranging from atom arrangements lacking any long-range order or lower level periodicity to regular single and multiple crystals, including nanocrystals and microcrystals, for example.

The atomic percentage of metal atoms in the material may suitably be in the range 1-100%.

Examples of suitable metal oxides includes silver species, such as silver(l) oxide and silver(I,III) oxide; copper species, including copper(III) oxide; gold species, including gold(II I) oxide; and zinc species, including zinc(IV) oxide. Preferably, the metal oxide is silver(l) oxide, silver(I,III) oxide, silver (II,III) oxide, silver(III) oxide or any structure of silver oxide incorporating a composition of oxygen that produces a silver release profile in aqueous media suitable for the chosen antibacterial application. More preferably, the metal oxide is silver(I,III) oxide, otherwise known as silver(II) oxide. More preferably still, the metal oxide is silver (I) oxide.

Suitable organic polyacids and derivatives include polymeric poly(carboxylic acids) and polymeric poly(carboxylic esters). Preferably, the species is a poly(acrylate) and/or poly(methacrylate).

Examples of poly(acrylate) and/or poly(methacrylate) species are the resulting polymers or copolymers of monomers of the type: 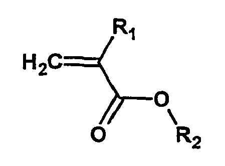 where
R₁ is methyl or hydrogen, and
R₂ is a straight- or branched-chain aliphatic hydrocarbyl group; such as C₁₋₆ alkyl, e.g. methyl,
C ₅₋₈ cycloalkyl, e.g. cyclohexyl;
araliphatic hydrocarbyl group, including a heteroaraliphatic hydrocarbyl group, optionally substituted in the aryl group, such as phenyl straight- and branched-chain C₁₋₆ alkyl, e.g. phenethyl, optionally substituted in the phenyl group; or
an optionally substituted aromatic hydrocarbyl group, including heteroaromatic hydrocarbyl groups, e.g. phenyl, optionally substituted in the phenyl group.
R₂ is preferably a straight chain or branched aliphatic moiety.

Preferably, the polyacid or derivative is a copolymer based on monomers that confer adhesive properties on it, allowing ready attachment to the metal oxide particles, which are often in dispersion in a fluid phase that comprises the adhesive species.

Most preferably, acrylate or methacrylate monomers are selected from a group comprising: acrylic acid, 2-ethylhexylacrylate, n-butylacrylate, 2-hydroxyethylmethacrylate and n-butylmethacrylate. Such monomers are often used as emulsions or solutions, resulting in an adhesive copolymer in dispersion, emulsion or solution.

The metal oxides can be combined with the acid or derivative by any method known to one skilled in the art that does not compromise the stability of the metal oxide.

Intimate combination of the metal oxide and polyacid or derivative can be effected by mechanical mixing and agitation of the two, preferably as a slurry of the metal oxide species with the polyacid or derivative in dispersion, emulsion or solution.

The metal oxides can be conveniently combined with the acid or derivative when the latter is an adhesive by mixing and agitation of a slurry of the metal oxide species in the polyacid or derivative adhesive formulation.

According to a third aspect of the present invention, there is provided a material for the treatment or prophylaxis of microbial, including bacterial, infections, characterised in that it comprises at least one Group IA or IB metal oxide and a polyphosphate salt and/or a reaction product of the metal oxide and the salt.

The materials of the first and third aspects of the present invention overcome the limitations of known antimicrobial, including antibacterial, materials. For example, they have a profile of release spanning several days. The materials exhibit a range of profile of release and delivery rate of the relevant active species, for example into aqueous media. The material compositions and components can be tailored to generate specific desired release rates, for example in aqueous media.

The quantity of silver that can be provided in a given mass of material is effectively diluted relatively little by the presence of the oxide ion or the polyphosphate.

The metal oxide-polyphosphate materials of the first or third aspects of this invention exhibit enhanced stability compared with that of the corresponding metal oxide alone. Articles comprising them can be stored for long periods (up to several years) at ambient temperature and pressure in traditional sterile packaging.

Particles in sizes ranging from atomic clusters to macroparticles, rather than individual metal atoms or ions within the metal oxide, can be readily stabilised with species that are not complex and/or costly.

Effectively a coating of the polyanion complex is formed on the surface of the metal oxide particles with species that are not complex and/or costly.

The metal oxide within the material will usually be in the form of particles in sizes ranging from atomic clusters, nanoclusters, colloids, aggregates, nanoparticles and microparticles, for example to macroparticles, with levels of structural order ranging from atom arrangements lacking any long-range order or lower level periodicity to regular single and multiple crystals including nanocrystals and microcrystals, for example.

The atomic percentage of metal atoms in the material may suitably be in the range 1-100%.

Examples of suitable metal oxides includes silver species, such as silver(I) oxide and silver(I,III) oxide; copper species, including copper(III) oxide; gold species, including gold(III) oxide; and zinc species, including zinc(IV) oxide.

Polyphosphates comprise more than one phosphate monomer moiety, and are able to exist as linear and branched polymeric chains and cyclic structures, and offer a 2-D and 3-D array of oxyanions of flexible geometry that can be accommodated in the surface structure of the metal oxide particles.

For atomic clusters, it is believed (without in any way limiting the present invention) that the precise atomic geometry of the metal oxide particle dictates the optimum size and geometry of the polyphosphate used for stabilisation.

Preferred silver oxide-polyphosphate materials of the first or third aspects of this invention with enhanced stability compared with that of the corresponding metal oxide alone and good antimicrobial, including antibacterial, efficacy include those in which the complexing polyphosphate contains more than two phosphate units.

Combination of metal oxide and polyphosphate can be achieved by any means known to the skilled person. Most conveniently, the metal oxide particles are presented in an aqueous medium for complexation with the polyphosphate in solution. For example, sodium polyphosphate solution may be added to silver(I,III) oxide or silver (I) oxide powder and the mixture homogenised by grinding.

Alternatively, metal oxide particles can be generated in situ, in the presence of polyoxyanions to generate the relevant metal oxide-polyphosphate complex. In this embodiment of the process, the polyphosphate geometry dictates to some extent the structure (and thus reactivity) of the so formed metal oxide particle. This is an example of a template synthesis.

This invention also relates to the use of polyphosphates for the stabilisation of metal cations, for example silver cations.

Experimental investigations have also shown that water-soluble polyphosphates stabilise metal cations, for example silver cations. These cations may be part of a cation-anion complex or be free of anions in solution, for example aqueous solution.

The stabilising polyphosphates are linear or branched water-soluble or water-semi-soluble polymers of the type:

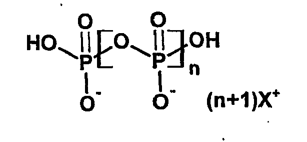

Where 'n' is an integer equal to or greater than 1. Preferably 'n' is an integer equal to or greater than 2. More preferably 'n' is an integer equal to or greater than 9.

The cation 'X' is not limited, but is preferably sodium.

Suitable polyphosphates therefore include: diphosphates, triphosphates, tetraphosphates, pentaphosphates, hexaphosphates and metaphosphates; for example, sodium hexametaphosphate, sodium triphosphate pentabasic, sodium pyrophosphate tetrabasic. Preferably, the polyphosphate is sodium hexametaphosphate.

'Silver cation' means a silver species that is electron deficient in comparison with a silver atom. The most common silver cation is Ag(1+) but the scope of this invention includes any silver cations, for example, Ag(1+), Ag(2+) and Ag(3+). Preferably, the silver cations are Ag(1+) or Ag(3+).

These silver cations can be part of an ionic complex with an anion or may be essentially anion-free , for example in solution. Common anions that form ionic complexes with silver cations include, but are not restricted to: acetate, acetylacetonate, arsenate, benzoate, bromate, bromide, carbonate, chlorate, chloride, chromate, citrate, cyanate, cyclohexanebutyrate, diethyldithiocarbamate, fluoride, heptafluorobutyrate, hexafluoroantimonate, hexafluoroarsenate, hexafluorophosphate, iodate, iodide, lactate, metavanadate, methanesulfonate, molybdate, nitrate, nitrite, oxide, pentafluoropropionate, perchlorate, permanganate, perrhenate, phosphate, phthalocyanate, selenide, sulfadiazine, sulfate, sulfide, sulfite, telluride, tetrafluoroborate, tetratungstate, thiocyanate, toluenesulfonate, trifluoroacetate and trifluoromethanesulfonate.

Preferably the anion is biologically acceptable, for example: nitrate, acetate or sulfadiazine.

For polyphosphate stabilisation in solution, solvated silver cations may be provided from a silver cation-anion complex, for example a simple salt such as silver(I) nitrate, silver sulfadiazine, silver phosphate or silver oxide. For medical applications, the solution is preferably aqueous, or partly aqueous.

Silver cations can be stabilised as part of an ionic complex or as essentially free cations in solution by water-soluble polyphosphates.

Combination of silver cations, including silver cations in ionic complexes, can be combined with polyphosphates by any means available to one skilled in the art. Most simply, silver cations or ionic silver complexes can be immersed in a solution of the stabilising phosphate, either permanently, or for a limited period of time.

According to a fourth aspect of the present invention, there is provided a composition for the treatment or prophylaxis of microbial, including bacterial, infections, comprising a material according to the first or third aspects of the present invention.

Preferably, the polymer acts as a barrier between the metal species and the remainder of the composition. Preferably, the metal species is less reactive with the polymer than with the remainder of the composition. Preferably, the polymer is less susceptible to oxidation by the metal species than the remainder of the composition.

In the first aspect of the present invention, suitable compositions include liquids, gels and creams for topical or internal administration per se or as a component of topical dressings, containing, e.g. the relevant metal oxide particles in dispersion in the fluid phase:

Suitable compositions also include surface-sterilising compositions, in particular for implantable devices, including long-term implants, such as artificial joints, fixation devices, sutures, pins or screws, catheters, stents, drains and the like.

In the first and third aspects of the present invention, suitable compositions include liquids, gels and creams for topical or internal administration per se or as a component of topical dressings, containing, e.g. the relevant metal oxide-polyphosphate complex particles in dispersion in the fluid phase, e.g. hydrogels and xerogels, e.g. cellulosic hydrogels, such as cross-linked carboxymethylcellulose hydrogels, for the management of wounds, including surgical, acute and chronic wounds, and burns, and surface-sterilising compositions, in particular for implantable devices, including long-term implants, such as artificial joints, fixation devices, sutures, pins or screws, catheters, stents, drains and the like.

According to a fifth aspect of the present invention, there is provided a device comprising a material according to the first or third aspects of the present invention.

According to a sixth aspect of the present invention, there is provided a device comprising a composition according to the fourth aspect of the present invention.

Preferably, the polymer acts as a barrier between the metal species and the device. Preferably, the metal species is less reactive with the polymer than with the device. Preferably, the polymer is less susceptible to oxidation by the metal species than the device.

Suitable devices include dressings, including topical dressings for the management of wounds, including surgical, acute and chronic wounds, and burns; implants including long-term implants, such as artificial joints, fixation devices, sutures, pins or screws, catheters, stents, drains and the like; artificial organs and scaffolds for tissue repair; and hospital equipment, such devices including, for example, operating tables.

Often the material of the first or third aspects of the present invention or a composition of the fourth aspect of the present invention is present as a coating on a surface of the medical device or a component thereof.

Suitable manufacturing methods are known to those skilled in the art and include dipping, fluid or powder coating and attachment via an adhesive or powder coating or blasting.

Such an adhesive coating which serves to attach the metal oxide species to the substrate on a surface of the medical device or a component thereof is advantageously robust, uniform and flexible on conformable substrates. Articles so produced can be stored for long periods, up to several years, at ambient temperature and pressure in traditional sterile packaging.

According to a seventh aspect of the present invention, there is provided a use in the treatment or prophylaxis of microbial, including bacterial, infections, comprising the use of a material according to the first or third aspects of the present invention, a composition according to the fourth aspect of the present invention, or a device according to the fifth or sixth aspect of the present invention.

Such a use in the treatment or prophylaxis of microbial, including bacterial, infections is useful in particular for the management of wounds, including surgical, acute and chronic wounds, and burns.

Reference will now be made, by way of example, to the accompanying drawings in which:
Figure 1 shows a material according to an embodiment of the present invention combined with a device; and
Figure 2 shows a material according to another embodiment of the present invention combined with a composition/device.

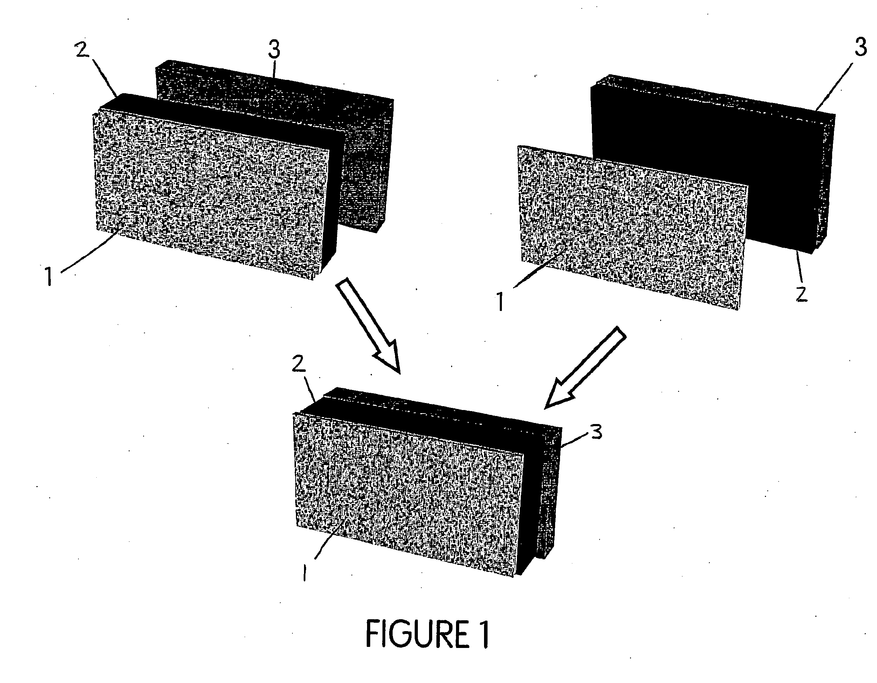

As shown in Figure 1, a layer comprising a metal species (1) is separated from a substrate (3) by a layer comprising a polymer (2). The polymer (2) is acting as a barrier between the metal species (1) and the substrate (3). The substrate (3) may be a medical device, for example.

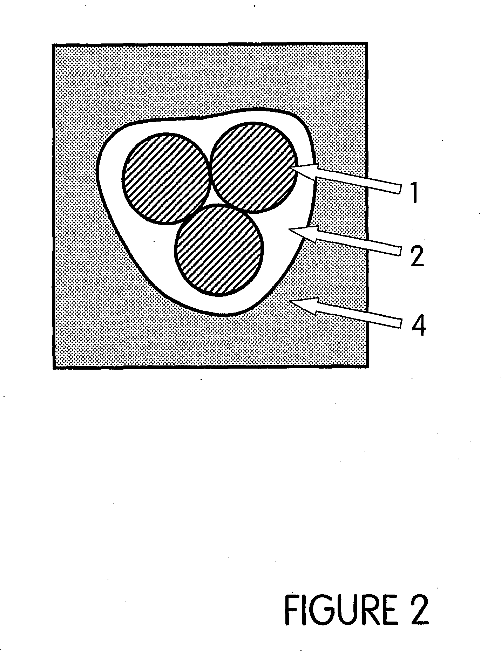

As shown in Figure 2, a metal species (1) is separated from a medium (4) by a polymer (2). As in Figure 1, the polymer (2) is acting as a barrier between the metal species (1) and the medium (4). The medium (4) may be a composition or a medical device.

In both Figures 1 and 2, the principle is the same. That is, the polymer (2) acts as a barrier between the metal species (1) and the substate (3) or medium (4) and thereby stabilises the metal species (1). Substrates (3)/media (4) that cause instability in ionic metal species are those susceptible to oxidation (i.e. electron donors). Such substrates include materials comprising electron-donating moieties, such as sulphur atoms, nitrogen atoms (including polyurethanes), aromatic species, unsaturated species and sugars (including polysaccharides). By providing a barrier between the metal species (1) and the substrate (3) or media (4), compositions or devices according to the present invention prevent instability in the metal species (1).

Materials and compositions according to the present invention provide effective barriers and therefore stabilise the metal species. Preferably, the metal species (1) is less reactive with the polymer (2) than with the substrate (3) or medium (4). Preferably, the polymer (2) is less susceptible to oxidation by the metal species (1) than the substrate (3) or medium (4).

The present invention is further illustrated by the following Examples relating to the first and second aspects of the present invention.

### EXAMPLE 1 FOR REFERENCE ONLY

### Silver(I,II) oxide-dispersion in adhesive

Silver(I,III) oxide (Aldrich Chemical Co.), 500 mg, was slurried in 6 ml distilled water prior to vigorous mixing into K5T adhesive emulsion (Smith & Nephew Medical Ltd.), 50 g resulting in a 1 %w/w composition. The grey coloured emulsion was spread onto acetate film via a 0.016 mm aperture spreading block and allowed to dry at ambient temperature and pressure. Special lighting precautions were not taken.
The resulting adhesive film was transparent grey in colour, with silver(I,III) oxide particles fully encapsulated within the adhesive.

### EXAMPLE 2 FOR REFERENCE ONLY

### Silver(I) oxide-dispersion in adhesive

Silver(I) oxide (Aldrich Chemical Co.), 500 mg, was slurried in 6 ml distilled water prior to vigorous mixing into K5T adhesive emulsion (Smith & Nephew Medical Ltd.), 50 g resulting in a 1%w/w composition. The grey coloured emulsion was spread onto acetate film via a 0.016 mm aperture spreading block and allowed to dry at ambient temperature and pressure. Special lighting precautions were not taken.

The resulting adhesive film was transparent brown in colour, with silver(I) oxide particles fully encapsulated within the adhesive.

### EXAMPLES FOR REFERENCE ONLY

The silver(I,III) oxide and silver(I) oxide adhesive films prepared in EXAMPLE 1 and EXAMPLE 2 were cut using a hydraulic press and cutting die. The cut sections of adhesive film were observed for 24 hours.

Within 2 hours of cutting, the brown film produced in EXAMPLE 2 had severely discoloured along the cut edge, with intense brown discolouration extending several millimetres into the adhesive film. The grey film produced in EXAMPLE 1 was unaffected by cutting.

### EXAMPLE 4 FOR REFERENCE ONLY

### Silver(I) oxide brushing onto A8 adhesive

The backing was removed from Opsite film (Smith & Nephew Medical Ltd.), exposing the adhesive contact layer. A small quantity of silver(I) oxide powder (Aldrich Chemical Co.), 200 mg, was centrally positioned and brushed onto the film using a standard bristle paint brush (ANZA woodstain and varnish brush, 1.5" round, 40 mm) until a uniform coating was achieved. Excess silver(I) oxide wash brushed from the surface.

### EXAMPLE 5 FOR REFERENCE ONLY

### Silver(I,III) oxide brushing onto A8 adhesive

The backing was removed from Opsite film (Smith & Nephew Medical Ltd.), exposing the adhesive contact slayer. A small quantity of silver(I,III) oxide powder (Aldrich Chemical Co.), 200 mg, was centrally positioned and brushed onto the film using a standard bristle paint brush (ANZA woodstain and varnish brush, 1.5" round, 40 mm) until a uniform coating was achieved. Excess silver(I,III) oxide wash brushed from the surface.

### EXAMPLE 6 FOR REFERENCE ONLY

### Silver (I,III) oxide brushing onto A8 and K5 adhesive

Double-side coated wound contact layer (Smith & Nephew Medical Ltd.), was exposed A8 adhesive side facing, with the reverse, K5-coated face, attached to release paper. A small quantity of silver(I,III) oxide powder (Aldrich Chemical Co.), 200 mg, was centrally positioned and brushed onto the adhesive film using a standard bristle paint brush (ANZA woodstain and varnish brush, 1.5" round, 40 mm) until a uniform coating was achieved. Excess silver(I,III) oxide wash brushed from the surface. The wound contact layer mesh was removed from the release paper backing and transferred, coated side down, onto release paper. The K5 adhesive surface was then coated with silver(I,III) oxide as above, resulting in a silver(I,III) oxide coated mesh.

### EXAMPLE 7 FOR REFERENCE ONLY

### Silver (I,III) oxide brushing onto wound contact layer mesh and transfer onto Allevyn foam

Double-side coated wound contact layer (Smith & Nephew Medical Ltd.), was exposed A8 adhesive side facing, with the reverse, K5-coated face, attached to release paper. A small quantity of silver(I,III) oxide powder (Aldrich Chemical Co.), 200 mg, was centrally positioned and brushed onto the adhesive film using a standard bristle paint brush (ANZA woodstain and varnish brush, 1.5" round, 40 mm) until a uniform coating was achieved. Excess silver(I,III) oxide wash brushed from the surface. The wound contact layer mesh was removed from the release paper backing and transferred, adhesive side down, coated side up, onto 6 mm thickness Allevyn foam.

### EXAMPLE 8 FOR REFERENCE ONLY

### Silver (I,III) oxide brushing onto wound contact layer mesh and transfer onto Allevyn foam and coated with wound contact layer

Double-side coated wound contact layer (Smith & Nephew Medical Lid.), was exposed A8 adhesive side facing, with the reverse, K5-coated face, attached to release paper. A small quantity of silver(I,III) oxide powder (Aldrich Chemical Co.), 200 mg, was centrally positioned and brushed onto the adhesive film using a standard bristle paint brush (ANZA woodstain and varnish brush, 1.5" round, 40 mm) until a uniform coating was achieved. Excess silver(I,III) oxide wash brushed from the surface. The wound contact layer mesh was removed from the release paper backing and transferred, adhesive side down, coated side up, onto 6 mm thickness Allevyn foam. The resulting composite was overplayed on the silver(I,III) oxide-coated face with a layer of double-sided adhesive wound contact layer, producing an adhesive foam device.

### EXAMPLE 9 FOR REFERENCE ONLY

### Silver (I,III) oxide brushing onto A8 adhesive and coating with wound contact layer

The backing was removed from Opsite film (Smith & Nephew Medical Ltd.), exposing the adhesive contact layer.

A small quantity of silver(I,III) oxide powder (Aldrich Chemical Co.), 200 mg, was centrally positioned and brushed onto the film using a standard bristle paint brush (ANZA woodstain and varnish brush, 1.5" round, 40 mm) until a uniform coating was achieved. Excess silver(I,III) oxide wash brushed from the surface. The resulting composite was overplayed on the silver(I,III) oxide-coated face with a layer of double-sided adhesive wound contact layer, producing an adhesive film device.

### EXAMPLE 10 FOR REFERENCE ONLY

The formats produced in EXAMPLES 1,3,5-9 were cut into 1 cm diameter circle and 5 x 5 cm square device formats by hydraulic press. None of the device formats discoloured on long standing at ambient temperature and pressure over several weeks. In comparison, controls lacking an adhesive barrier coating, directly exposing the silver oxide to the polyurethane substrate, discoloured within 24 hours with concomitant loss of antimicrobial efficacy.

The present invention is further illustrated by the following Examples relating to the first and third aspects of the present invention.

### EXAMPLE 11

### Silver(I) oxide and sodium polyphosphate combination and dispersion in cross-linked carboxymethylcellulose hydrogel

A solution of sodium polyphosphate (Aldrich Chemical Co.), 0.14 g, was prepared in distilled water, 2 ml. This solution was added to silver(I) oxide powder (Aldrich Chemical Co.), 0.14 g, in a marble mortar and the mixture was homogenised by pestle grinding.

The silver(I) oxide-polyphosphate material was added to IntraSite gel (Smith & Nephew Medical Ltd), 14.00 g, and mixed mechanically.

### EXAMPLE 12

### Silver(I,III) oxide* and sodium polyphosphate combination and dispersion in cross-linked carboxymethylcellulose hydrogel

A solution of sodium polyphosphate (Aldrich Chemical Co.), 0.14 g, was prepared in distilled water, 2 ml. This solution was added to silver(I,III) oxide powder (Aldrich Chemical Co.), 0.14 g, in a marble mortar and the mixture was homogenised by pestle grinding. The silver(I,III) oxide-polyphosphate material was added to IntraSite gel (Smith & Nephew Medical Ltd), 14.00 g, and mixed mechanically.
*Otherwise known as silver(II) oxide.

### EXAMPLE 13

### Gold(III) oxide and sodium polyphosphate combination and dispersion in cross-linked carboxymethylcellulose hydrogel

A solution of sodium polyphosphate (Aldrich Chemical Co.), 0.14 g, was prepared in distilled water, 2 ml. This solution was added to gold(III) oxide powder (Aldrich Chemical Co.), 0.14 g, in a marble mortar and the mixture was homogenised by pestle grinding. The gold(III) oxide-polyphosphate material was added to IntraSite gel (Smith & Nephew Medical Ltd), 14.00 g, and mixed mechanically.

### EXAMPLE 14

### Stabilisation of Gold(III) oxide formulation in aqueous polysaccharide gel with sodium hexametaphosphate

Gold(III) oxide (1g) was suspended in 10 ml aqueous solution containing sodium hexametaphosphate (1 g). The suspension was vigourously mixed into 100 g Intrasite Gel (Smith & Nephew Medical Ltd). The resulting mixture was stored at 20°C in the absence of light. A control was prepared lacking the sodium hexametaphosphate. This was stored in identical conditions. After 6 months, each sample was examined. The sample including sodium hexametaphosphate was unchanged in appearance and viscosity while the sample lacking sodium hexametaphosphate had changed colour and reduced significantly in viscosity, indicating that significant degradation had occurred.

### EXAMPLE 15

### Stability comparison of metal oxide and sodium polyphosphate combinations and controls lacking polyphosphate stabilisation

The preparations in EXAMPLES 11-13 were repeated in the absence of sodium polyphosphate. Metal oxide-matched pairs were observed for 24 hours after preparation.

It was observed that, in the absence of sodium polyphosphate, the metal oxide-hydrogel combinations discoloured within hours of preparation, indicating decomposition. In contrast, those formulations including sodium polyphosphate did not discolour within 24 hours. Furthermore, these formulations did not discolour within 14 days.

### EXAMPLE 16

### Antimicrobial activity of metal oxide-polyphosphate dispersions in cross-linked carboxymethylcellulose hydrogel

The compositions prepared in EXAMPLES 11-13 were tested for antimicrobial activity against two bacterial strains:

Pseudomonas aeruginosa NCIMB 8626 and Staphylococcus aureus NCTC 10788 were harvested. Serial 1:10 dilutions were performed to give a final concentration of 10⁸ bacteria/ml. Further dilutions were made for an inoculum count, down to 10⁻⁸ bacteria/ml, with the number of bacteria/ml determined using the pour plate method.

Two large assay plates were then set up and 140 ml of Mueller-Hinton agar was added evenly to the large assay plates and allowed to dry (15 minutes). A further 140 ml of agar was seeded with the corresponding test organism and poured over the previous agar layer. Once the agar had set (15 minutes), the plate was dried at 37°C for 30 minutes with the lid removed. 8 mm plugs were removed from the plate by biopsy punch.

In triplicate, the compositions prepared in EXAMPLES 11-13 and controls prepared in EXAMPLE 15 were transferred by 3 ml capacity syringe into holding cups having a 6 mm diameter aperture cut in the agar-contacting surface. The plates were then sealed and incubated at 37°C for 24 hours. The size of the bacterial zone cleared was measured using a Vernier calliper gauge, triplicates were averaged.

### EXAMPLE 17

### Stabilisation of silver ions formulated in aqueous polysaccharide gel with sodium hexametaphosphate

Silver nitrate (1g) was dissolved in 10 ml aqueous solution containing sodium hexametaphosphate (1g). The resulting opaque suspension was vigourously mixed into 100 g Intrasite Gel (Smith & Nephew Medical Ltd). The resulting mixture was stored at 20°C in the absence of light. A control was prepared lacking the sodium hexametaphosphate. This was stored in identical conditions. After 24 hours, each sample was examined. The sample including sodium hexametaphosphate was unchanged in appearance and viscosity while the sample lacking sodium hexametaphosphate had changed colour to dark brown and increased significantly in viscosity, indicating that significant degradation had occurred.

## Claims

1. A material for the treatment or prophylaxis of microbial, including bacterial, infections comprising a metal species and a polymer, wherein the polymer is a polyanion which stabilises the metal species, **characterised in that** the polyanion is a polyphosphate.

2. A material according to claim 1, wherein the polyphosphate is selected from the group consisting of diphosphates, triphosphates, tetraphosphates, pentaphosphates and metaphosphates.

3. A material according to claim 2, wherein polyphosphate is sodium hexametaphosphate.

4. A material according to any of claims 1-3, wherein the polymer is a reaction product of the metal species and the polyphosphate and/or a derivative thereof.

5. A material according to any preceding claim, wherein the metal species is selected from the group consisting of silver, copper, zinc, manganese, gold, iron, nickel, cobalt, cadmium, palladium and platinum species.

6. A material according to any preceding claim, wherein the metal species is selected from the group consisting of metal ion, metal salt, metal cluster, metal particle, metal nanoparticle and metal crystal.

7. A material according to any preceding claim, wherein the metal species is a metal oxide.

8. A material according to any preceding claim, wherein the metal species is a silver species.

9. A material according to any preceding claim, wherein the metal species is a silver cation.

10. A material according to claim 8, wherein the silver species is selected from the group consisting of silver nitrate, silver perchlorate, silver acetate, silver tetrafluoroborate, silver triflate, silver fluoride, silver chloride, silver oxide and silver hydroxide.

11. A material according to claim 10, wherein the silver oxide is selected from the group consisting of silver (I) oxide, silver (I,III) oxide, silver (II,III) oxide, and silver (III) oxide.

12. A material according to any of claims 1 to 7, wherein the metal species is a gold species.

13. A material according to claim 12, wherein the gold species is selected from the group consisting of gold nitrate, gold perchlorate, gold acetate, gold tetrafluoroborate, gold triflate, gold fluoride, gold chloride, gold oxide and gold hydroxide.

14. A composition for the treatment or prophylaxis of microbial, including bacterial, infections, comprising a material according to any of claims 1 to 13.

15. A composition according to claim 14, wherein the polyphosphate acts as a barrier between the metal species and the remainder of the composition.

16. A composition according to claim 15, wherein the metal species is less reactive with the polyphosphate than with the remainder of the composition.

17. A composition according to claim 15 or 16, wherein the polyphosphate is less susceptible to oxidation by the metal species than the remainder of the composition.

18. A device comprising a material according to any of claims 1 to 13.

19. A device according to claim 18, wherein the polyphosphate acts as a barrier between the metal species and the device.

20. A device according to claim 18 or 19, wherein the metal species is less reactive with the polyphosphate than with the device.

21. A device according to any of claims 18 to 20, wherein the polyphosphate is less susceptible to oxidation by the metal species than the device.

22. A device comprising a composition according to any of claims 14 to 17.

## Patentansprüche

1. Ein Material zur Behandlung oder Prophylaxe mikrobieller, einschließlich bakterieller, Infektionen, das eine Metallspezies und ein Polymer beinhaltet, wobei das Polymer ein Polyanion ist, welches die Metallspezies stabilisiert, **dadurch gekennzeichnet, dass** das Polyanion ein Polyphosphat ist.

2. Material gemäß Anspruch 1, wobei das Polyphosphat aus der Gruppe, bestehend aus Diphosphaten, Triphosphaten, Tetraphosphaten, Pentaphosphaten, Hexaphosphaten und Metaphosphaten, ausgewählt ist.

3. Material gemäß Anspruch 2, wobei das Polyphosphat Natriumhexametaphosphat ist.

4. Material gemäß einem der Ansprüche 1-3, wobei das Polymer ein Reaktionsprodukt der Metallspezies und des Polyphosphats und/oder eines Derivats davon ist.

5. Material gemäß einem der vorhergehenden Ansprüche, wobei die Metallspezies aus der Gruppe, bestehend aus Silber-, Kupfer-, Zink-, Mangan-, Gold-, Eisen-, Nickel-, Cobalt-, Cadmium-, Palladium- und Platinspezies, ausgewählt ist.

6. Material gemäß einem der vorhergehenden Ansprüche, wobei die Metallspezies aus der Gruppe, bestehend aus Metallion, Metallsalz, Metallcluster, Metallpartikel, Metallnanopartikel und Metallkristall, ausgewählt ist.

7. Material gemäß einem der vorhergehenden Ansprüche, wobei die Metallspezies ein Metalloxid ist.

8. Material gemäß einem der vorhergehenden Ansprüche, wobei die Metallspezies eine Silberspezies ist.

9. Material gemäß einem der vorhergehenden Ansprüche, wobei die Metallspezies ein Silberkation ist.

10. Material gemäß Anspruch 8, wobei die Silberspezies aus der Gruppe, bestehend aus Silbernitrat, Silberperchlorat, Silberacetat, Silbertetrafluorborat, Silbertriflat, Silberfluorid, Silberchlorid, Silberoxid und Silberhydroxid, ausgewählt ist.

11. Material gemäß Anspruch 10, wobei das Silberoxid aus der Gruppe, bestehend aus Silber(I)-oxid, Silber(I,III)-oxid, Silber(II,III)-oxid und Silber(III)-oxid, ausgewählt ist.

12. Material gemäß einem der Ansprüche 1 bis 7, wobei die Metallspezies eine Goldspezies ist.

13. Material gemäß Anspruch 12, wobei die Goldspezies aus der Gruppe, bestehend aus Goldnitrat, Goldperchlorat, Goldacetat, Goldtetrafluorborat, Goldtriflat, Goldfluorid, Goldchlorid, Goldoxid und Goldhydroxid, ausgewählt ist.

14. Eine Zusammensetzung zur Behandlung oder Prophylaxe mikrobieller, einschließlich bakterieller, Infektionen, die ein Material gemäß einem der Ansprüche 1 bis 13 beinhaltet.

15. Zusammensetzung gemäß Anspruch 14, wobei das Polyphosphat als eine Barriere zwischen der Metallspezies und dem Rest der Zusammensetzung wirkt.

16. Zusammensetzung gemäß Anspruch 15, wobei die Metallspezies gegenüber dem Polyphosphat weniger reaktiv als gegenüber dem Rest der Zusammensetzung ist.

17. Zusammensetzung gemäß Anspruch 15 oder 16, wobei das Polyphosphat gegen Oxidation durch die Metallspezies weniger empfindlich als der Rest der Zusammensetzung ist.

18. Eine Vorrichtung, die ein Material gemäß einem der Ansprüche 1 bis 13 beinhaltet.

19. Vorrichtung gemäß Anspruch 18, wobei das Polyphosphat als eine Barriere zwischen der Metallspezies und der Vorrichtung wirkt.

20. Vorrichtung gemäß Anspruch 18 oder 19, wobei die Metallspezies gegenüber dem Polyphosphat weniger reaktiv als gegenüber der Vorrichtung ist.

21. Vorrichtung gemäß einem der Ansprüche 18 bis 20, wobei das Polyphosphat gegen Oxidation durch die Metallspezies weniger empfindlich als die Vorrichtung ist.

22. Eine Vorrichtung, die eine Zusammensetzung gemäß einem der Ansprüche 14 bis 17 beinhaltet.

## Revendications

1. Un matériau destiné au traitement ou à la prophylaxie d'infections microbiennes, y compris bactériennes, comprenant une espèce métallique et un polymère, dans lequel le polymère est un polyanion qui stabilise l'espèce métallique, **caractérisé en ce que** le polyanion est un polyphosphate.

2. Un matériau selon la revendication 1, dans lequel le polyphosphate est sélectionné dans le groupe consistant en diphosphates, triphosphates, tétraphosphates, pentaphosphates, hexaphosphates et métaphosphates.

3. Un matériau selon la revendication 2, dans lequel le polyphosphate est de l'hexamétaphosphate de sodium.

4. Un matériau selon n'importe lesquelles des revendications 1 à 3, dans lequel le polymère est un produit de la réaction de l'espèce métallique et du polyphosphate et / ou un dérivé de celui-ci.

5. Un matériau selon n'importe quelle revendication précédente, dans lequel l'espèce métallique est sélectionnée dans le groupe consistant en espèces argent, cuivre, zinc, manganèse, or, fer, nickel, cobalt, cadmium, palladium et platine.

6. Un matériau selon n'importe quelle revendication précédente, dans lequel l'espèce métallique est sélectionnée dans le groupe consistant en ion métallique, sel métallique, amas métallique, particule métallique, nanoparticule métallique et cristal métallique.

7. Un matériau selon n'importe quelle revendication précédente, dans lequel l'espèce métallique est un oxyde métallique.

8. Un matériau selon n'importe quelle revendication précédente, dans lequel l'espèce métallique est une espèce argent.

9. Un matériau selon n'importe quelle revendication précédente, dans lequel l'espèce métallique est un cation argent.

10. Un matériau selon la revendication 8, dans lequel l'espèce argent est sélectionnée dans le groupe consistant en nitrate d'argent, perchlorate d'argent, acétate d'argent, tétrafluoroborate d'argent, triflate d'argent, fluorure d'argent, chlorure d'argent, oxyde d'argent et hydroxyde d'argent.

11. Un matériau selon la revendication 10, dans lequel l'oxyde d'argent est sélectionné dans le groupe consistant en oxyde d'argent (I), oxyde d'argent (I, III), oxyde d'argent (II, III), et oxyde d'argent (III).

12. Un matériau selon n'importe lesquelles des revendications 1 à 7, dans lequel l'espèce métallique est une espèce or.

13. Un matériau selon la revendication 12, dans lequel l'espèce or est sélectionnée dans le groupe consistant en nitrate d'or, perchlorate d'or, acétate d'or, tétrafluoroborate d'or, triflate d'or, fluorure d'or, chlorure d'or, oxyde d'or et hydroxyde d'or.

14. Une composition destinée au traitement ou à la prophylaxie d'infections microbiennes, y compris bactériennes, comprenant un matériau selon n'importe lesquelles des revendications 1 à 13.

15. Une composition selon la revendication 14, dans laquelle le polyphosphate fait fonction de barrière entre l'espèce métallique et le reste de la composition.

16. Une composition selon la revendication 15, dans laquelle l'espèce métallique est moins réactive avec le polyphosphate qu'avec le reste de la composition.

17. Une composition selon la revendication 15 ou la revendication 16, dans laquelle le polyphosphate est moins susceptible d'être oxydé par l'espèce métallique que le reste de la composition.

18. Un dispositif comprenant un matériau selon n'importe lesquelles des revendications 1 à 13.

19. Un dispositif selon la revendication 18, dans lequel le polyphosphate fait fonction de barrière entre l'espèce métallique et le dispositif.

20. Un dispositif selon la revendication 18 ou la revendication 19, dans lequel l'espèce métallique est moins réactive avec le polyphosphate qu'avec le dispositif.

21. Un dispositif selon n'importe lesquelles des revendications 18 à 20, dans lequel le polyphosphate est moins susceptible d'être oxydé par l'espèce métallique que le dispositif.

22. Un dispositif comprenant une composition selon n'importe lesquelles des revendications 14 à 17.
